Europäisches Patentamt

(19) European Patent Office

Office européen des brevets

(11) Veröffentlichungsnummer: **0 160 689**
**B1**

(12) **EUROPÄISCHE PATENTSCHRIFT**

(45) Veröffentlichungstag der Patentschrift:
**22.06.88**

(51) Int. Cl.⁴: **A 61 B 17/36**

(21) Anmeldenummer: **84904093.6**

(22) Anmeldetag: **29.10.84**

(86) Internationale Anmeldenummer:
**PCT/DE 84/00227**

(87) Internationale Veröffentlichungsnummer:
**WO 85/01870 (09.05.85 Gazette 85/11)**

(54) NEODYM-YAG-LASER INSBESONDERE ZUR OPHTALMOLOGISCHEN BEHANDLUNG.

(30) Priorität: **29.10.83 DE 3339369**

(43) Veröffentlichungstag der Anmeldung:
**13.11.85 Patentblatt 85/46**

(45) Bekanntmachung des Hinweises auf die Patenterteilung:
**22.06.88 Patentblatt 88/25**

(84) Benannte Vertragsstaaten:
**AT BE CH DE FR GB LI NL SE**

(56) Entgegenhaltungen:
**DE - A - 2 713 903**
**US - A - 3 817 604**
**US - A - 4 203 652**

**Laserfocus, Band 19, Januar 1983 (US), "NdiYAG Lasers in opthalmic surgery", Seite 38**

(73) Patentinhaber: **MEDITEC REINHARDT THYZEL GMBH, Obere Bergstrasse 3, D-8501 Heroldsberg (DE)**

(72) Erfinder: **SCHRÖDER, Eckhard, Hans Sachsstr. 9, D-8501 Eckental (DE)**
Erfinder: **THYZEL, Reinhardt, Obere Bergstr. 3, D-8501 Heroldsberg (DE)**

(74) Vertreter: **Steinmann, Otto C. et al, Kanzlei Münich, Steinmann, Schiller Willibaldstrasse 36, D-8000 München 21 (DE)**

**Beschreibung**

1. Die Erfindung bezieht sich auf einen Neodym-YAG-Laser insbesondere zur ophtalmologischen Behandlung gemäss dem Oberbegriff des Patentanspruchs 1.

Neodym-YAG-Laser gemäss dem Oberbegriff des Patentanspruchs 1 werden beispielsweise zu Operationen am menschlichen Auge eingesetzt. Da bei derartigen Operationen der sog. optische Durchbruch grösser als der Laserstrahlfleck ist, ist bisher der Variation der Fleckgrösse und damit auch der damit verbundenen Variation des Winkels der Randstrahlen des fokussierten Strahls, d.h. der Variation des sog. Divergenzwinkels keine Beachtung geschenkt worden.

Die bekannten Neodym-YAG-Laser sind deshalb so aufgebaut, dass der Laserstrahl mit einem bestimmten festen Divergenzwinkel fokussiert wird.

Erfindungsgemäss ist jedoch erkannt worden, dass aus Sicherheitsgründen eine Variation des Winkels der Randstrahlen des fokussierten Strahls, d.h. eine Variation des Divergenzwinkels wünschenswert ist: der Divergenzwinkel sollte nämlich möglichst gross sein, damit die Energiedichte des Operationsstrahls vor und nach der Arbeitsebene im Auge, auf die der Laser fokussiert ist, schnell abnimmt.

Andererseits ist der Winkel der Randstrahlen durch die Grösse der Pupille begrenzt. Um immer mit dem maximal möglichen Divergenzwinkel arbeiten zu können, wäre also eine Variation dieses Winkels und damit verbunden eine Variation der Fleckgrösse des Fokus erforderlich. Dabei soll die Verschiebung der Fokusebene des fokussierten Strahls möglichst gering sein.

Bei Argonlasern ist es bekannt, die Fleckgrösse ohne Verschiebung der Fokusebene mit Hilfe einer Zoomoptik zu verstellen, die sehr aufwendig und teuer ist.

Eine derartige Zoomoptik ist bei Neodym-YAG-Lasern nicht nur aus Kostengründen, sondern auch noch aus folgendem Grunde nicht wünschenswert: Bei Verwendung von Neodym-YAG-Lasern als Operationslaser, deren Licht nicht sichtbar ist, wird häufig zusätzlich ein Helium-Neon-Laser als Ziellaser verwendet. Da auch der Strahl des Helium-Neon-Lasers in der selben Ebene wie der Strahl des Neodym-YAG-Lasers fokussiert werden soll, ist es erforderlich, die Zoomoptik über einen weiten Bereich sehr gut farbzukorrigieren, so dass ihr Aufbau zwangsläufig sehr kompliziert sein muss.

Der Erfindung liegt die Aufgabe zugrunde, eine einfache optische Strahlführung für einen Neodym-YAG-Laser zu schaffen, die eine Verstellung des Divergenzwinkels ohne spürbare Verschiebung der Fokusebene erlaubt.

Diese Aufgabe wird erfindungsgemäss ausgehend von einem Neodym-YAG-Laser mit einer Strahlführung gemäss dem Oberbegriff des Patentanspruchs 1 durch die im kennzeichnenden Teil des Patentanspruchs 1 angegebenen Merkmale gelöst.

Erfindungsgemäss ist erkannt worden, dass bei den meisten zu opthalmologischen Behandlungen eingesetzten Neodym-YAG-Lasern der Laserstrahl aus irgendwelchen Gründen – beispielsweise zur Einspiegelung in den Strahlengang einer Spaltlampe – über eine grosse Strecke – vielfach von mehr als einem Meter – geführt wird, und erst dann in das menschliche Auge fokussiert wird.

Bei einem Neodym-YAG-Laser mit einer derartigen Strahlführung kann der Divergenzwinkel leicht dadurch verstellbar ausgeführt werden, dass in den Parallelstrahlengang in einem ausreichenden Abstand vor der Fokussieroptik ein afokales System eingebracht wird. Dieses afokale System verändert in seiner Grundstellung das parallele Strahlenbündel, d.h. den Durchmesser dieses Strahlenbündels praktisch nicht. Zum Vergrössern bzw. zum Verkleinern des Divergenzwinkels genügt es, eines der beiden optischen Glieder dieses zusätzlich eingebrachten Systems geringfügig aus der Grundstellung, in der dieses System afokal ist, zu verschieben. Durch diese kleine Verschiebung eines der beiden Glieder wird das parallele Strahlenbündel nicht mehr in ein paralleles Strahlenbündel abgebildet, vielmehr entsteht aus dem parallelen Strahlenbündel ein geringfügig konvergentes oder divergentes Strahlenbündel. Durch die grossen, über ca. 0,5 m liegenden Strecken, ergibt sich aufgrund der winzigen Winkeländerung eine starke Aperturvergrösserung und damit eine grosse Variation des Divergenzwinkels sowie eine Variation der Fleckgrösse. Die Verschiebung der Fokusebene liegt dabei innerhalb der Schärfentiefe des Messmikroskops, ist also zu vernachlässigen.

In den Unteransprüchen sind vorteilhafte Ausgestaltungen der Erfindung angegeben.

Das optische Glied, das zur Variation des Winkels der Randstrahlen dient, kann kontinuierlich verschiebbar sein, so dass sich der Divergenzwinkel kontinuierlich über einen weiten Bereich einstellen lässt.

In der Praxis ist aber oft die leichter zu realisierende Ausbildung gemäss Anspruch 2 ausreichend, nach der lediglich drei Stellungen des den Divergenzwinkel ändernden optischen Gliedes vorgesehen sind: eine mittlere für normales Arbeiten, eine Stellug, in der der Divergenzwinkel gegenüber der mittleren Stellung verkleinert ist, und eine Stellung für grosse Divergenzwinkel, die allerdings auch grosse Pupillenöffnungen erfordert.

In jedem Falle ist es vorteilhaft, wenn die Verschiebung des optischen Glieds motorisch erfolgt, da insbesondere bei Augenuntersuchungsgeräten der Neodym-YAG-Laser und die zur Divergenzwinkelbeeinflussung vorgesehenen optischen Bauteile nicht in Griffweite der Bedienungsperson angeordnet sind. Derartige motorische Verstellungen, die beispielsweise Mikroschalter oder andere Bauteile zum Erfassen der Stellung des verschiebbaren optischen Gliedes aufweisen, sind in der Technik bekannt, so dass auf ihre Realisierung nicht näher eingegangen werden muss.

Gemäss Anspruch 4 ist vor dem afokalen System ein teildurchlässiger oder ein wellenlängenselektiver Spiegel angeordnet, mit dem der Laserstrahl eines Helium-Neon-Lasers, der als Zielstrahl in an sich bekannter Weise verwendet wird, einspiegelbar ist.

Besonders überraschend ist es, dass das afokale System ein aus zwei einzellinsen bestehendes System sein kann, das nicht nur den Strahl des Neodym-YAG-Lasers mit einer Wellenlänge von 1,06 μm, sondern auch den Strahl des Helium-Neon-Lasers mit einer Wellenlänge von 633 nm auch ohne weitere Korrektur ohne Verschiebung der Fokussierebene abbildet.

Das in Anspruch 5 gekennzeichnete weitere optische System weitet den Strahlengang auf den geforderten Arbeitsdurchmesser erst auf, nachdem der Strahlengang über eine grosse Entfernung als paralleles oder leicht divergentes bzw. konvergentes Bündel mit einem verhältnismässig kleinen Durchmesser geführt worden ist. Hierdurch kann der Durchmesser der Laserstrahl-Führung wesentlich herabgesetzt werden.

Besonders überraschend ist, dass ein einfaches nur aus zwei Linsen bestehendes System auch den Helium-Neon-Zielstrahl ohne weitere Korrektur und ohne Verschiebung der Fokusebene abbildet.

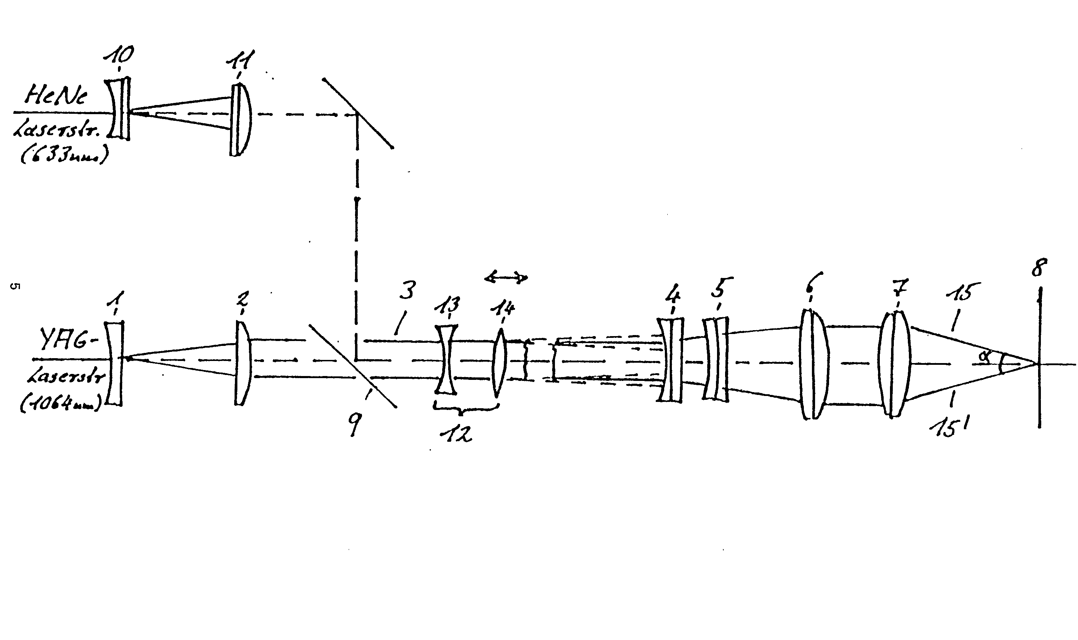

Die Erfindung wird nachstehend anhand eines Ausführungsbeispiels unter Bezugnahme auf die Zeichnung näher beschrieben, deren einzige Figur den Strahlengang eines erfindungsgemässen Neodym-YAG-Lasers bei Verwendung als Operationslaser mit einem Helium-Neon-Zielstrahllaser zeigt.

Der Laserstrahl des Neodym-YAG-Lasers wird von den Linsen 1 und 2 aufgeweitet und als paralleles Strahlenbündel 13 über eine grössere Entfernung, beispielsweise eine Entfernung von 2 m geführt. Danach wird der Strahl von den optischen Gliedern 4, 5 und 6, die zur Farbkorrektur achromatische Kittglieder sind, nochmals aufgeweitet, und anschliessend von dem Kittglied 7 auf die Arbeitsebene 8, beispielsweise den Augenhintergrund eines menschlichen Auges fokussiert.

Im Bereich der ersten parallelen Strahlenführung 3 des Yag-Laserstrahl wird mittels eines teildurchlässigen bzw. farbselektiven Spiegels 9 der von Kittglieder 10 und 11 aufgeweitete Strahl eines Helium-YAG-Lasers eingespiegelt, der als Zielstrahl verwendet wird. Dabei wird unter farbselektiver Spiegel ein Spiegel verstanden, der Licht der Wellenlänge des Neodym-YAG-Lasers (1064 nm) durchlässt, Licht der Wellenlänge des Helium-Neon-Lasers (633 nm) aber reflektiert.

Soweit die in der Figur dargestellte optische Anordnung vorstehend beschrieben worden ist, ist sie bekannt und wird beispielsweise beim YAG-Laser OPL 4 der Meditec Reinhardt Thyzel GmbH, 8501 Heroldsberg, Deutschland, verwendet. Auf die Ausbildung sowie die Verwendungsmöglichkeiten dieses bekannten Lasers wird ausdrücklich Bezug genommen.

Erfindungsgemäss ist in dem Bereich der parallelen Strahlführung 3 des YAG-Laserstrahls zwischen dem Spiegel 9 und dem Kittglied 4 mit einem Abstand von mehr als 50 cm von dem Glied 4 ein afokales System 12 eingebracht, das aus einer positiven Linse 13 und einer negativen Linse 14 besteht. In seiner Grundstellung verändert das afokale System 12 den parallelen Strahlengang, d.h. den Durchmesser des parallelen Strahlenbündels praktisch nicht.

Durch eine Verschiebung entweder der positiven Linse 13 oder der negativen Linse 14 wird das parallele Strahlenbündel in ein geringfügig konvergentes oder divergentes Bündel abgebildet. Aufgrund der grossen Strecke, die das Strahlenbündel vor der nochmaligen Aufweitung und Fokussierung zurückzulegen hat, ergeben winzigste Winkeländerungen eine grosse Aperturvergrösserung oder -verkleinerung und damit eine grosse Änderung des Divergenzwinkels, d.h. des von den Randstrahlen 15 und 15′ des fokussierten Strahls eingeschlossenen Winkels $\alpha$. Die Fokusänderung ist dabei zu vernachlässigen und liegt innerhalb der Schärfentiefe des Messmikroskops für den Zielvorgang, mit dem die Arbeitsebene beispielsweise im menschlichen Auge überprüft wird.

Die nochmalige Aufweitung des Strahls durch die Glieder 4, 5 und 6 hat dabei einen positiven Einfluss auf die bei gegebener Entfernung erreichbare Divergenzwinkelvariation.

Dabei ist der Fachmann ohne weiteres in der Lage, beispielsweise bei vorgegebenem Durchmesser des Parallelstrahlenbündels und Abstand Fokussierlinsengruppe/Arbeitsebene unter Einhaltung von Randbedingungen wie Länge des Strahlengangs das afokale System und die zur Herbeiführung einer bestimmten Änderung des Divergenzwinkels erforderlichen Verschiebungen eines Gliedes dieses Systems zu berechnen, so dass auf die Angabe von numerischen Daten verzichtet werden kann.

Darüber hinaus eignet sich das erfindungsgemäss vorgesehene System auch zur Änderung der Fleckgrösse: durch eine geringfügige Defokussierung des Laserstrahls und durch Ändern des Divergenzwinkels kann der Durchmesser des Strahls beispielsweise in der Ebene des Augenhintergrundes variiert werden. Die Durchmesseränderung kann dabei leicht mittels des Helium-Neon-Ziellaserstrahls überprüft werden.

## Patentansprüche

1. Neodym-YAG-Laser insbesondere zur ophtalmologischen Behandlung, mit einer Laserstrahlführung, in der der Laserstrahl über eine grössere Strecke als paralleles Strahlenbündel geführt ist, und mit einer Fokussieroptik, die im Anschluss an diese Strecke den Laserstrahl auf die Arbeitsebene fokussiert, dadurch gekennzeichnet, dass in den Parallelstrahlengang (3) mit einem Abstand von mehr als ca. 50 cm von der Fokussieroptik (7) ein afokales System (12) mit mehreren optischen Gliedern (13, 14) eingebracht ist, das in seiner Grundstellung das parallele Strahlenbündel praktisch nicht verändert, und in dem ein optisches Glied (13 oder 14) zur Variation des Winkels ($\alpha$) der

Randstrahlen (15, 15') des fokussierten Strahls verschiebbar ist.

2. Laser nach Anspruch 1, dadurch gekennzeichnet, dass drei Stellungen für das zur Variation des Winkels ($\alpha$) der Randstrahlen (15, 15') verschiebbare optische Glied (13 oder 14) vorgesehen sind.

3. Laser nach Anspruch 1 oder 2, dadurch gekennzeichnet, dass zur Verschiebung des optischen Glieds (13 oder 14) ein Motor mit einer Steuereinheit vorgesehen ist.

4. Laser nach einem der Ansprüche 1 bis 3, dadurch gekennzeichnet, dass vor dem afokalen System ein teildurchlässiger oder wellenlängenselektiver Spiegel (9) angeordnet ist, mit dem ein Helium-Neon-Laserstrahl zur Verwendung als Zielstrahl einspiegelbar ist.

5. Laser nach einem der Ansprüche 1 bis 4, dadurch gekennzeichnet, dass das afokale System aus zwei Einzellinsen (13, 14) besteht, von denen eine positive und die andere negative Brechkraft hat, und von denen eine verschiebbar ist.

6. Laser nach einem der Ansprüche 1 bis 5, dadurch gekennzeichnet, dass ein weiteres optisches System den Strahlengang nach dem afokalen System nochmals aufweitet.

## Claims

1. A neodymium YAG laser, especially for ophthalmological treatment, comprising a laser beam control system wherein the laser beam is guided as a beam of parallel rays over a major distance, and comprising an optical focussing system to focus the laser beam onto the plane of operation joining said distance, characterized in that an afocal system (12) comprising several optical elements (13, 14) is introduced into the path of parallel rays (3) at a distance of more than 50 cm approximately from the focussing system (7), which afocal system, in its initial position, does practically not change the beam of parallel rays, and in which system an optical element (13 or 14) is displaceable so as to vary the angle ($\alpha$) of the fringe beams (15, 15') of the focussed beam.

2. A laser system in accordance with claim 1, characterized in that three positions are provided for the optical element (13 or 14) displaceable so as to vary the angle ($\alpha$) of the fringe beams (15, 15').

3. A laser system in accordance with Claim 1 or 2, characterized in that a motor with a controller unit is provided for the displacement of said optical element (13 or 14).

4. A laser system in accordance with any of Claims 1 through 3, characterized in that a partially reflecting end plate or an end plate selective in terms of wavelength (9) is arranged ahead of the afocal system, which is suited for superimposition by way of reflexion of a helium-neon laser beam for application as the collimation beam.

5. A laser system in accordance with any of Claims 1 through 4, characterized in that said afocal system is composed of two individual lenses (13, 14) of which one has a power of converging refraction while the other has a power of diverging refraction, and whereof one is displaceable.

6. A laser system in accordance with any of Claims 1 through 5, characterized in that the path of rays undergoes a further expansion in a supplementary optical system joining said afocal system.

## Revendications

1. Laser YAG au néodyme, en particulier pour le traitement opthalmologique, à un système de focalisation du faisceau laser dans lequel le faisceau laser est guidé comme un faisceau parallèle par une distance majeure, et à un système optique de focalisation qui en suite à cette distance focalise le faisceau laser sur le plan d'opération, caractérisé en ce qu'un système afocal (12) à plusieurs éléments optiques (13, 14) est disposé dans la marche des rayons parallèles (3) à une distance de plus de 50 cm environ du système optique de focalisation (7), ledit système afocal pratiquement ne variant, dans sa position initiale, le faisceau parallèle, et dans lequel système afocal un élément optique (13 ou 14) est déplaçable afin de varier l'angle ($\alpha$) des rayons marginaux (15, 15') du faisceau focalisé.

2. Laser selon la revendication 1, caractérisé en ce que trois positions sont pourvues pour l'élément optique (13 ou 14) déplaçable pour la variation de l'angle ($\alpha$) des rayons marginaux (15, 15').

3. Laser selon la revendication 1 ou 2, caractérisé en ce qu'un moteur à un organe de commande est pourvu pour le déplacement de l'élément optique (13 ou 14).

4. Laser selon quelconque des revendications 1 à 3, caractérisé en ce qu'en amont du système afocal, un miroir semi-argenté ou sélectif en vue de longueur d'onde (9) est disposé qui est apte à superposer par réflexion un faisceau laser à hélium et néon pour l'application comme faisceau de collimation.

5. Laser selon quelconque des revendications 1 à 4, caractérisé en ce que le système afocal est composé par deux lentilles individuelles (13, 14) dont l'une a une force de réfraction convergente et l'autre a une force de réfraction divergente, et dont une est déplaçable.

6. Laser selon quelconque des revendications 1 à 5, caractérisé en ce que la marche des rayons subit un autre élargissement dans un système optique supplémentaire en aval du système afocal.